(19) Europäisches Patentamt
European Patent Office
Office européen des brevets

(11) Publication number: 0 076 478
B1

(12) # EUROPEAN PATENT SPECIFICATION

(45) Date of publication of patent specification: 15.01.86

(51) Int. Cl.[4]: C 12 Q 1/00, G 01 N 33/84

(21) Application number: 82109065.1

(22) Date of filing: 01.10.82

(54) **Linear kinetic determination of bicarbonate in body fluids.**

(30) Priority: 05.10.81 US 308284

(43) Date of publication of application:
13.04.83 Bulletin 83/15

(45) Publication of the grant of the patent:
15.01.86 Bulletin 86/03

(84) Designated Contracting States:
AT BE CH DE FR GB IT LI LU NL SE

(56) References cited:
US-A-3 974 037

CLINICAL CHEMISTRY, vol. 22, no. 2, 1976, R.L. FORRESTER et al. "Enzymatic method for determination of CO2 in serum", pages 243 to 245

THE JOURNAL OF BIOLOGICAL CHEMISTRY, vol. 246, no. 17, 1971, T.G. COOPER et al. "The carboxylation of phosphoenolpyruvate and pyruvate", pages 5488 to 5490

CLINICAL CHEMISTRY, vol. 25, no. 4, 1979, R. REJ "Measurement of aspartate aminotransferase activity: Effects of oxamate", pages 555 to 559

(73) Proprietor: BOEHRINGER MANNHEIM CORPORATION
P.O. Box 50 100, 9115 Hague Road
Indianapolis Indiana 46250 (US)

(72) Inventor: Peled, Nina
7830 Oakington Drive
Houston Texas 7707 (US)

(74) Representative: Weber, Manfred, Dr. et al
Boehringer Mannheim GmbH Patentabteilung
Sandhofer Strasse 116
D-6800 Mannheim 31 (DE)

EP 0 076 478 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

This invention relates to enzymatic determinations of the bicarbonate content in a sample of a body fluid, for example in blood plasma or serum, and more particularly to determinations which couple the reaction for carboxylation of phosphoenolpyruvate in the presence of phosphoenolpyruvate carboxylase to the oxidation reaction for the reduced form of nicotinamide adenine dinucleotide by oxaloacetate in the presence of malate dehydrogenase.

Phosphoenolpyruvate carboxylase [EC 4.1.1.31] catalyzes the fixation of carboxylation of phosphoenolpyruvate by the reaction:

$$PEP + HCO_3^- \xrightarrow[Mg^{2+}]{PEPC} OAA + Pi$$

where "PEP" is phosphoenolpyruvate, "$HCO_3^-$" is bicarbonate, "PEPC" is phosphoenolpyruvate carboxylase, "$MG^{2+}$", is divalent, magnesium cation, "OAA" is oxaloacetate and "Pi" is inorganic orthophosphate. (For simplicity this reaction hereinafter is referred to as "reaction I", and these same abbreviations are sometimes used hereinafter in the text as well as in reaction formulas, a convention with respect to other abbreviations which is used hereinafter).

Oxaloacetate, a reactant in the Krebs or citric acid cycle of metabolism, is converted to malate by the reduced form of the coenzyme nicotinamide adenine dinucleotide in the presence of malate dehydrogenase [EC 1.1.1.37], according to the following reaction ("reaction II"):

$$OAA + NADH \xrightarrow{MDH} Malate + NAD^+$$

where "MDH" is malate dehydrogenase, "NADH" is the reduced form of nicotinamide adenine dinucleotide, and "$NAD^+$" is the oxidized form of nicotinamide adenine dinucleotide. In an enzymatic reaction in which NADH is oxidized to $NAD^+$, the amount of NADH oxidized will be, mole for mole, equivalent to corresponding substrate conversion or product formation. In reaction II, for every mole of NADH oxidized to $NAD^+$, one mole of OAA is converted to malate.

The level of absorbance of a compound is a measure of the concentration of that compound in a solution. The coenzyme nicotinamide adenine dinucleotide absorbs ultraviolet (UV) light in its reduced state (NADH) between about 320 nm (nanometers) and 400 nm, the absorbance peak being about 340 nm. Absorbance of the oxidized state of nicotinamide adenine dinucleotide ($NAD^+$) is negligible at these spectra. Accordingly, the amount of NADH oxidized in a reaction is conveniently measured by following the decrease in absorbance of the reaction solution at a selected wavelength (preferably 340 nm or thereabout) within the range from 320 nm to 400 nm.

Nicotinamide adenine dinucleotide is not the coenzyme for all clinically important enzyme reactions, but it can be and often is linked or coupled with other enzymatic reactions which are of principal interest (the "primary reaction") to follow substrate conversion or product formation taking placing in the primary reaction. Here, the product of the primary reaction is used as the substrate in the coupled or "secondary" enzymatic reaction for which the coenzyme NADH is required. This has long been a technique for measurements in respect to reaction I. See, e.g., Tchen, T. T. and Vennesland, B., "Enzymatic Carbon Dioxide Fixation into Oxalacetate in Wheat Germ", *J. Biol. Chem.,* Vol. 213, pp. 533—546) (1955); Bandurski, R. S., "Further Studies on the Enzymatic Synthesis of Oxalacetate from Phosphoenolpyruvate and Carbon Dioxide", *J. Biol. Chem.,* Vol. 217, pp. 137—150 (1955); Maruyama, H., Easterday, H., Chang, H., and Lane, M. D. "The Enzymatic Carboxylation of Phosphoenolpyruvate", *J. Biol. Chem.,* Vol. 241, pp. 2405—2412 (1966); Smith, T. E., "Escherichia Coli Phosphoenolpyruvate Carboxylase: Characterization and Sedimentation Behavior", *Arch. Bioch. and Biophys.,* Vol. 128, pp. 611—622 (1968); and Cooper, T. G. and Wood, H. G., "The Carboxylation of Phosphoenolpyruvate and Pyruvate", *J. Biol. Chem.,* Vol. 246, pp. 5488—5490 (1971).

Bicarbonate is the second largest fraction of the ions in blood serum or plasma. The determination of the bicarbonate content of blood serum or plasma is a significant indicator of electrolyte dispersion and anion deficit. Knowledge of the bicarbonate content (and pH) of blood serum or plasma assists in the medical diagnosis of acid-base imbalance in the respiratory and metabolic systems.

In recent years there have been efforts to determine body fluid bicarbonate by enzymatic procedures coupling reactions I and II. See, for example, Wilson, W., Jesyk, P., Rand, R. and Bevill, R. D., *Clin. Chem.,* Vol. 19, p. 640 (1973); Menson, R. C., Narayanswany, V., Bussian, R. W., and Adams, T. H., *Clin. Chem.,* Vol. 20, p. 872 (1974); U.S. Patent 3,974,037, and Forrester, R. L., Wataji, L. J., Silverman D. A., and Pierre, K. J., *Clin. Chem.,* Vol. 22, pp. 243—245 (1976).

The enzymes operating in coupled and secondary reactions are merely biological catalysts. They increase the rate of a reaction without being consumed in the reaction or appearing as reaction products. Generally speaking, they speed up reactions by more efficiently organizing the substrate so that less

energy is required for the reaction to proceed. An enzyme does not determine the quantity of end product which the reaction will form; it only determines the rate at which the product is formed, i.e., the quantity of product formed within a period of time. At a specific level of substrate, the amount of end product formed will remain the same at various enzyme activities, but that amount of end product will be formed in a shorter time as enzyme activity increases. The concentration of enzyme in a solution is defined in terms of activity units. The unit of activity is the measure of the rate at which the reaction proceeds, derived from the quantity of substrate consumed or product formed in a convenient unit of time at a specified temperature.

In enzyme catalyzed reactions the concentration of a substrate may be determined by allowing the reaction to go to completion or by a rate determination method. See, e.g., *Principles of Enzymatic Analysis*, Hans Ulrich Bergmeyer Ed., Verlag Chemie, New York (1978), p. 40, p. 81. Substrate concentration with a reaction allowed to go to completion may be measured, for example, in a secondary NADH reaction coupled to a primary reaction, by the total decrease in NADH absorbance in the secondary reaction over the period of reaction time taken for the speed of the absorbance-decrease to diminish to an insignificant rate. At this "end-point", effectively all the quantity of product which the primary reaction will form (under the reaction conditions) has been produced, and so, effectively all the substrate for that amount of product has been converted. Since there is a mole for mole correspondence between both NADH converted to $NAD^+$ (in the coupled reaction) and substrate converted to product (in the primary reaction), the total change in NADH absorbance indicates the amount of substrate consumed in the primary reaction. In clinical chemistry assays of the content of bicarbonate in a body fluid such as blood serum or plasma, an end-point determination requires a relatively large ratio of the reagent volume (which contains the PEP, PEPC, NADH and MDH) to blood serum or plasma sample volumes, as well as a spectrophotometric instrument which can read high absorbances. For example, for a sample bicarbonate content of 40 milliequivalents per liter, where two milliliters of reagent are combined with 10 microliters of sample, the absorbance change would be 1.24 absorbance units. These requirements are not acceptable for many automated clinical analyzers.

Instead of an end-point measurement to determine the content of a substrate present in a sample such as blood serum, the content may be determined as a function of how rapidly the reaction converting the substrate to product proceeds. This "rate determination" or "kinetic" method depends on the principle that the rate of the reaction will be proportional to the concentration of a substrate converted by the reaction, where the concentration of the substrate limits the rate of the reaction. The basis of this principle is that, in an enzymatic reaction, the rate at which a substrate is converted to product depends both upon the basic physical rate of the enzyme catalyzed reaction and the frequency at which enzyme molecules and substrate molecules collide. The rate of collision is proportional to the concentration of substrate molecules. As substrate concentration is increased, frequency of collision reaches a maximum and is no longer a factor in determining reaction rate. At this substrate concentration, the concentration of substrate does not limit the rate at which the reaction can enzymatically proceed; only enzyme activity determines the rate of conversion of substrate molecules to product molecules. thus, in a rate determination of the content of a substrate in a sample, all other substrates participating in the reaction must be present at concentrations which do not limit the reaction rate, so that, insofar as substrates are concerned, only the concentration of the tested substrate is "rate-limiting". Accordingly, in the use of reaction I to kinetically determine bicarbonate in a sample, only the bicarbonate should be rate-limiting; PEP must be provided in amounts which do not limit the rate at which PEPC catalyzes the carboxylation of PEP to OAA. Where reaction I is linked to reaction II, NADH also must be provided in "non-rate-limiting amounts", and activity units of MDH must be sufficiently greater than the activity units of the PEPC employed so as to catalyze the rate of reaction II at a speed no slower than the rate of reaction I. Then, since the rate of formation of the OAA limits the rate at which NADH is converted to $NAD^+$, and since the rate of formation of OAA is in turn dependent upon the concentration of bicarbonate in the sample, spectrophotometric measurement of units of NADH absorbance-decrease within a fixed-time interval (i.e., the rate of absorbance change) can be used to determine the bicarbonate concentration in the sample.

In order to correlate a determined rate of absorbance change to the concentration of the substrate at which that rate occurs, it must be known whether the rate of absorbance change is directly proportional, or proportional in a more complex way, to the substrate concentration. This is learned by repetitively conducting the same test, using the same reagents under the same conditions, and making the rate of absorbance change measurements at the same fixed-time interval, but varying the concentration (known) of the substrate. For each substrate concentration used, the absorbance change corresponding to concentration is plotted (on the y-axis) against substrate concentration (on the x-axis). If the relationship between rate of absorbance change for the fixed-time interval and substrate concentration is linear from substantially the coordinates zero/zero to the maximum substrate concentration which may be determined, then only the rate of absorbance change for one known substrate concentration need to be known in order to determine an unknown substrate concentration for which a rate of absorbance change is obtained (under the same conditions and same fixed-time interval). But if the plotted relationship between rate of absorbance change and substrate concentration is not linear, resort must be made to the curve described by such relationship in order to determine an unknown substrate concentration corresponding to a measured rate of absorbance change. The curve so employed is called a "standard curve". Prior methods employing reactions I and II for a rate determination of bicarbonate in a body fluid (as exemplified by U.S. Patent 3,974,037) have required generation and utilization of a standard curve, for in them the relationship

between the rate NADH absorbance change and bicarbonate concentrations is not linear for all bicarbonate concentrations.

Moreover, prior rate determinations for bicarbonate in body fluid using a reagent for reactions I and II may yield falsely elevated results. Blood serum usually contains endogenous pyruvate and lactate dehydrogenase [EC 1.1.1.27]. In the presence of NADH provided in the reagent for reaction II, NADH is converted to $NAD^+$ by the reaction ("reaction III"):

$$\text{Pyruvate}+\text{NADH} \xrightarrow{\text{LDH}} \text{Lactate}+\text{NAD}^+$$

where "LDH" is lactate dehydrogenase. Since bicarbonate content of the body fluid is determined by following the decrease in NADH absorbance, the conversion of NADH caused by reaction III and consequent decrease in NADH absorbance is misread as a decrease in NADH absorbance caused by reaction II, giving the false appearance that more OAA was produced in reaction I (and, therefore, that more bicarbonate was present) than actually was; in short, the test results are falsely elevated. This interference with accuracy can be substantial. Hansen, N. Q., and Yasmineh, W. G., *Clin. Chem.,* Vol. 25, p. 1159 (1979) reported that a specimen of patient in septic shock was found to have a pyruvate concentration of 1.4 mmol/l and an LDH activity of 3000 units/l.

It is possible to eliminate this source of error by combining the body fluid with the buffer to be used for reactions I and II, and then "preincubating" the solution so that reaction III goes to completion before the assay reaction for bicarbonate is started (by adding the remainder of the ingredients used in the reagent). Exogenous LDH may be added to increase the rate of pyruvate conversion during the preincubation period. See, Liese, W., Jung, K., Ludtke, B., and Grutzmann, K. D., *Zentralbl. Pharm.,* Vol. 114, p. 1051—1064 (1975). This corrective measure, however, requiring preincubation, reading an initial absorbance at the end of the preincubation period, and then adding ingredients which start the assay reaction for bicarbonate, is impracticable with many automated analyzers.

A second possible cause of falsely high results [see, e.g., Bergmeyer, H. U. Scheibe, P. and Wahlefeld, A. W., *Clin. Chem.,* Vol. 24, pp. 58—73 (1978), and Rej, R. and Vanderline, R. E., *Clin. Chem.,* Vol. 20, pp. 454—464 (1974)] may be enzymatic (microorganisms) or nonenzymatic decarboxylation of OAA to pyruvate, according to the reaction

$$\text{OAA}\rightarrow\text{Pyruvate}+\text{CO}_2$$

The $CO_2$ formed by this reaction re-enters the reaction process as bicarbonate (reaction I), and the pyruvate reacts (reaction III) with NADH in the presence of LDH; as a consequence two equivalents of NADH are converted to $NAD^+$ for one OAA derived from bicarbonate originally present in the sample. The extent of carboxylation can be expected to be negligible, because OAA is in a steady-state low concentration and the reaction is quickly completed. But adding exogenous LDH as suggested above would only accentuate this second cause of falsely elevated results.

The invention

The "normal" concentration range for bicarbonate in blood serum or plasma is from about 22 to about 32 meq/l. Under abnormal or blood acid-base imbalance conditions, the bicarbonate ion concentration can range as low as 15 meq/l to as high as 40 meq/l. It is an object of my invention to provide means by which the bicarbonate concentration of a body fluid can be determined kinetically as a linear function of all bicarbonate concentrations which may occur under normal and abnormal conditions.

It is an object of this invention also to improve the accuracy of rate determinations for bicarbonate made according to this invention by eliminating the effect which endogenous LDH can have on endogenous pyruvate. In a one substrate reaction, i.e., where only one substrate is rate limiting, the reaction rate is described by the well known Michaelis-Menten equation

$$v=\frac{V[S]}{Km+[S]}$$

where v is the actual reaction rate, V is the maximum reaction rate, Km is the Michaelis-Menten constant of the enzyme for the substrate, and [S] is the substrate concentration. For the reaction rate to be a linear function of the substrate concentration, the substrate concentration ([S]) must be negligible with respect to the Michaelis-Menten constant (Km).

The Km reported by Smith, *supra*, for PEPC from *E. coli* (bicarbonate ion as the substrate) is $1.75\times10^{-3}$ moles per liter. For a serum sample which contains 40 meq/l of bicarbonate, using 2.5 ml of reagent (for reactions I and II) and 20 microliters of sample (see U.S. Pat. 3,974,037), the bicarbonate concentration in the reaction mixture would be $3.2\times10^{-4}$ moles per liter. Thus, the condition that the substrate concentration be negligible with respect to the Km is not satisfied. This explains the non-linearity observed with prior rate measurements employing reactions I and II when correlating the rate results with substrate

concentration, and the constraint associated therewith that a standard curve be generated in order to use reactions I and II to kinetically determine bicarbonate. This constraint imposed by the curvilinear reaction kinetics involved in prior enzymatic rate determinations for bicarbonate in body fluid is eliminated by my invention.

In accordance with my invention, there is provided a reagent which with respect to the volume of a reaction mixture formed upon combining a predetermined volume of a body fluid and a predetermined volume of the reagent, comprises inorganic pyrophosphate ($PPi^{4-}$") in combination with (i) nonrate-limiting amounts of PEP and NADH, (ii) activity units of PEPC sufficient to catalyze the formation of OAA from the PEP and bicarbonate in the reaction mixture at a rate suitable for fixed-time interval absorbance measurements of the reaction mixture, and (iii) activity units of MDH sufficient to catalyze the formation of malate from the NADH and the OAA at a rate no slower than the rate of formation of the OAA. The $PPi^{4-}$ is used in an amount, relative to the amount of PEP, effective to cause abnormal elevated concentrations of bicarbonate which could be present in the reaction mixture to be negligible with respect to the apparent bicarbonate Michaelis-Menten constant of the PEPC employed.

The known rate equation for a reaction influenced by a competitive inhibitor is:

$$v=\frac{V[S]}{Km(1+[I]/Ki)+[S]}$$

where the symbols V, [S] and Km have the meaning stated hereinbefore, [I] is the concentration of the competitive inhibitor and Ki is the dissociation constant for the enzyme complex resulting from combination of the enzyme and the inhibitor. This equation is seen to differ from the Michaelis-Menten equation only by the term (1+[I]/Ki). This term, in the equation (a) above, is multiplied by Km; the product Km(1+[I]/Ki) is the "apparent Michaelis-Menten constant" of the PEPC employed ((bicarbonate substrate). In my invention, by utilizing $PPi^{4-}$ as a competitive inhibitor in an amount selected in respect to the nonrate-limiting amount of PEP employed, the greatest projectable concentration of bicarbonate in the reaction mixture that will occur (under abnormal conditions in the subject from whom the body fluid sample is taken) will be negligible in relation to the apparent bicarbonate Michaelis-Menten constant for the PEPC employed, and the rate of reaction is thereby made linear, i.e., directly proportional, to all concentrations of bicarbonate which could be in the body fluid sample.

Maruyama et al., *supra*, examined the pH optimum of PEPC (extracted from peanut cotyledons) with phosphate buffer and Tris ($Cl^-$) buffer, and observed inorganic phosphate dianion to be inhibitory to PEPC, attributing this to phosphate dianion competition with the PEP phosphoryl dianion on its enzyme-binding site. In this connection, they noted orthophosphate dianion produced by reaction I with Tris ($Cl^-$) buffer (pH 7.8) to be apparently competitive with PEP ("product inhibition"), for the inhibition could be rerersed by increasing the PEP concentration. Accordingly, Maruyama et al. resorted to a non-phosphate buffer, as apparently subsequent investigators have, when employing reactions I and II. Phosphate buffers are also contraindicated for use with reactions I and II because, in the presence of magnesium and ammonium salts, formation of magnesium ammonium phosphate precipitants can complicate procedures employing enzymes suspended in saturated ammonium sulfate.

Apparently at least in part because of these factors, the tetra-anionic $PPi^{4-}$ has never been used to intentionally inhibit PEPC activity in a rate determination employing reactions I and II, much less in amounts — with respect to nonrate-limiting amounts of PEP — effective to transform the rate of reaction, from curvilinear for bicarbonate substrate concentrations expectable in a body fluid, to linear for all bicarbonate concentrations which may be expected to occur (under normal and abnormal conditions).

In order to increase the accuracy of linear rate determinations for body fluid bicarbonate according to my invention, the error source created by conversion of endogenous pyruvate to lactate by reaction III is substantially eliminated by adding a soluble salt of oxamic acid ("oxamate"), e.g. sodium oxamate, to the components which with the sample of body fluid will form the reaction mixture. Oxamate forms a ternary LDH-NADH-oxamate complex strongly inhibiting reaction III. Concentrations of oxamate up to 40 mmol/l have no effect on either PEPC of MDH. Suitably from about 5 to about 40 mmol/l, preferably from about 10 to about 20 mmol/l of oxamate, are employed. Oxamate is a known LDH inhibitor [Novoa, W. B., Winer, A. D. Glaire, A. J., and Schwert, G. W., *J. Biol. Chem.,* Vol. 234, pp. 1143—1148 (1959)] and has been used to inhibit endogenous LDH catalyzed conversion of endogenous pyruvate in body fluid assayed for asparate aminotransferase activity [see Trivedi, R. C., Strong, L, J., Dickstein, R., and Desai, K., *Clin. Chem.,* Vol. 23, p. 1171 (1977), and Lustig, V. and Redman, L. W., *Clin. Chem.,* Vol. 24, p. 989 (1978); cf. Rej. R., *Clin. Chem.,* Vol. 25, p. 555 (1979]. Oxamate has not been used, however, to increase the accuracy of bicarbonate determinations, much less in the presence of $PPi^{4-}$ in a rate determination for body fluid bicarbonate with reactions I and II.

In practice, to enable bicarbonate in a sample of body fluid to be determined by rate measurement, the volume of the sample and the concentration of bicarbonate in the sample for normal and abnormal conditions are established and the final volume of the reaction mixture resulting from combination of the sample and the reaction ingredients is set. For this volume activity units of PEPC are employed which, in relation to expectable maximum concentration of sample bicarbonate in the reaction mixture, will provide

a rate for reaction I suitable for fixed-time interval absorbance measurements of the reaction mixture. Since the rate of reaction I is measured by the rate of absorbance decrease of the reaction mixture corresponding to conversion of NADH to $NAD^+$ in reaction II, at least activity units of PEPC are provided sufficient to permit precise absorbance measurements taking into account the signal noise level of the electronics of the spectrophotometer employed, yet not so much as to cause reaction I to go to completion before at least one set of fixed-time interval measurements of NADH absorbance decrease can be taken, manually or according to the limiting characteristics of recording automation which may be employed. Fixed-time interval measurements, as is conventional, will be taken, while conducting the reactions at a substantially constant pH and temperature, after the start up "lag phase" of reaction I (when the reaction rate is increasing) and before bicarbonate depletion sets in (when the reaction rate is decreasing), i.e., when the reaction is proceeding at a constant rate. Since for a rate determination the duration of the fixed-time intervals will be selected for convenient measurement of reaction-mixture absorbance at the beginning and end of the interval, according to the capabilities of the apparatus used, and preferably, will be selected so as to allow a plurality of sets of fixed-time interval measurements to be taken (for averaging), the maximum activity units of PEPC employed to affect the rate of reaction I will be in reference to the limitations of the apparatus employed for fixed-time interval absorbance measurements. It is convenient usually to utilize activity units of PEPC which will control the rate of reaction I such that the duration selected for the fixed-time interval for absorbance measurement will be short with respect to the time it takes for reaction I, under the conditions used, to go to completion. For example, but not by way of limitation, if the reaction is established to go to completion in about twenty minutes after combination of the sample and the reaction ingredients, a 30-to-60-second fixed-time interval is short.

An amount of PEP is employed which will not limit the rate of reaction I. Conventionally a non-rate-limiting amount is at least 20 times the substrate Km for the enzyme, here the PEP Km of PEPC. Reaction II must not proceed at a rate slower than reaction I, so MDH is provided in activity units substantially in excess of the activity units of PEPC used. Suitably this may be a ratio of five activity units of MDH per activity unit of PEPC. A nonrate-limiting amount of NADH is also used but in reference to the measurable absorbance limit of the spectrophotometer to be employed. Normally an optical density of 2.0 is the measurable limit in a spectrophotometer.

In accordance with my invention, from about 0.2 to about 0.8 moles of $PPi^{4-}$ per mole of PEP are suitably employed, preferably from about 0.4 to about 0.6 moles, and most preferably a ratio of 0.51 moles of $PPi^{4-}$ per mole of PEP, to cause the expectable amounts of bicarbonate in the reaction mixture to be negligible with respect to the apparent Michaelis-Menten constant for the PEPC employed. Soluble $PPi^{4-}$ salts suitably employable for this purpose include disodium dihydrogen pyrophosphate, tetrapotassium pyrophosphate, tetra sodium pyrophosphate and dicalcium pyrophosphate.

PEPC is widely distributed in plant tissues and microorganisms, including spinach leaves [Bandwuski, R. S., and Greiner, C. M., *J. Biol. Chem., 204,* 781 (1953)], wheat germ [Tchen, T. T., and Vennesland, B., *J. Biol. Chem., 213,* 533 (1955)], peanut cotyledons [Manruyama, H. and Lane, M. D., *Biochem. Biophys, Acta, 65,* 207 (1962)], potatoe tubers, pea, and maize [Norris, K. A., Atkinson, A. R., and Smith, W. G., *Clin. Chem., 21,* 1093 (1975)], *Thiobacillus Thiooxidans* [Suzuki, I., and Werkman, C. H., *Arch. Biochem., Biophys. Acta, 96,* 169 (1965)] and *Escherichia coli* [Canovas, J. L., and Kornberg, H. L., *Biochem. Biophys. Acta, 96,* 169 (1965)], and is now commercially available, as is MDH.

Magnesium ion is well known as a cofactor for PEPC activity. PEPC from *E. coli* is activated by acetyl-coenzyme A but PEPC from wheat germ does not require acetyl-coenzyme A. These and other such factors affecting selection of the PEPC to be employed and its activity, as well as selection of a suitable basic pH within the range from about 7 to about 9.5, and a reaction temperature within the range generally from 20°C to 50°C, for conducting reactions I and II, are taught in the open literature, including the articles cited in the immediately foregoing paragraph and the articles by Smith, T. E., *Arc. Bioch. and Biophys.,* Vol. 128, pp. 611—622 (1968) and by Cooper, T. G. and Wood, H. G., *J. Biol. Chem.,* Vol. 246, pp. 5488—5490 (1971), which are incorporated hereby by reference. Preferably a reagent formulation according to my invention contains from about 3 to about 40 mmol/l of PEP, from about 0.2 to about 0.6 mmol/l of NADH, from about 0.1 to about 0.8 U/mol of PEPC, from about 0.5 to about 4 U/ml of malate dehydrogenase, from about 1 to about 30 mmol/l of $PPI^{4-}$, from about 0.5 to about 5 mmol/l magnesium ion, and a buffer having a pKa effective to provide a basic pH in the range from about 7 to 9.5. More preferably, the reagent also contains from about 5 to about 40 mmol/l of oxamate. Buffers having a pKa effective to control the pH of the reagent of a basic pH in the range from about 7 to about 9.5 include without limitation tris (hydroxymethyl) amino methane; N - tris - (hydroxymethyl - piperazine - N′ - 2 - ethane - sulfonic acid); and N - 2 - hydroxyethyl - piperazine - N′ - 3 - propanesulfonic acid. Suitably from about 5 to about 100 mmol/l of buffer may be used.

The bicarbonate in a sample of body fluid is determined by forming the reaction mixture as I have described (the "test reaction mixture") and also forming a second reaction mixture by combining the reagent I have described with a solution containing a known concentration of bicarbonate. Measurements of the decrease in absorbance of each of these mixtures is made after the same lapsed time (marked from when the reaction mixture is formed) at the same wavelength within the range from about 320 nm to about 400 nm and at the same fixed-time interval, while maintaining each such mixture at a substantially constant selected temperature and basic pH. There is then applied, to the measured absorbance decrease of the test

reaction mixture, the factor in which the numerator is the known bicarbonate concentration of the solution used to form the second reaction mixture and in which the denominator is the measured absorbance decrease of the second reaction mixture.

It is well known that at a basic pH, the equilibrium

$$H_2O+CO_2 \rightleftharpoons H_2CO_3 \rightleftharpoons H^+ + HCO_3^-$$

results in conversion of carbon dioxide to bicarbonate, and this is particularly so as bicarbonate is removed in reaction I by PEPC action, causing the equilibrium to move in the direction of bicarbonate formation. Since atmospheric $CO_2$ can react with the water in the reagent solution to form bicarbonate, a reagent blank is preferably employed in the rate determination of bicarbonate in a body fluid.

Use of a reagent blank entails exposing the reagent to the same temperature and pH conditions and for the same time as for the test reaction mixture formed by combining the reagent and a sample of the body fluid, while taking absorbance readings on the reagent at the same wavelength and at the same lapsed time and same fixed-time intervals at which absorbance readings are taken on the test reaction mixture. Any absorbance change caused by bicarbonate present in the reagent (as a result of the reaction of carbon dioxide with the water of the reagent) is subtracted from each of the absorbance changes measured for the reaction mixture and for the second mixture formed by combining my reagent with a solution of known bicarbonate concentration, described in the next preceding paragraph. The concentration of bicarbonate in the sample of body fluid is then determined by applying to the so reduced (or "net") measured absorbance decrease of the test reaction mixture, the factor in which the numerator is the known bicarbonate concentration of the said solution and in which the denominator is the so reduced (or "net") measured absorbance decrease of the said second reaction mixture.

Care should be taken to minimize the presence of dissolved carbon dioxide, so that "blank" readings are not so elevated as to significantly reduce spectrophotometer sensitivity. Thus, reagent components should be dissolved in freshly deionized or distilled water.

Example I

A reagent kit was formulated of two components, a buffer-substrate component and an enzymes suspension component. The buffer-substrate component contained

| | |
|---|---|
| PEP | 32 mmol/l |
| Sodium pyrophosphate | 15 mmol/l |
| NADH | 0.32 mmol/l |
| Magnesium acetate | 1.5 mmol/l |
| Tris-base (pH 8.0) | 10 mmol/l |
| Sodum oxamate | 10 mmol/l |

where mmol/l is millimoles per liter. NADH and PEP were obtained from Boehringer Mannheim Corporation, Indianapolis, Indiana; sodium pyrophosphate, from J. T. Baker Chemical Company, Phillipsburg, N.J.; and tris (hydroxymethyl) amino methane ("Tris-base") and sodium oxamate, from Sigma Chemical Co., St. Louis, Missouri.

The enzymes component, obtained from Bio-Research Products, Inc., Manhatten, Kansas, contained suspended in 50% saturated ammonium sulphate

| | |
|---|---|
| PEPC (wheat germ) | 12.0 U/ml |
| MDH (porcine heart) | 60.0 U/ml |

where one U/ml is an enzyme activity unit which catalyzes the transformation of one micromole per milliliter of substrate per minute at 37°C.

A pooled human serum sample containing 41.2 meq/l (milliequivalents per liter) sodium bicarbonate was serially diluted to provide 13 serum samples having the bicarbonate contents shown in Table I, below, under the caption "Bicarbonate-expected". From the enzyme suspension component of the reagent kit, 100 microliters were added to 1.5 ml of the buffer-substrate component to form the working reagent. The working reagent was vortexed and preincubated at 37°C for three minutes. Twenty-five microliters of one of the prepared serum samples was then added to the working reagent to form a reaction mixture, and the decrease in absorbance of the reaction mixture at 340 nm at 37°C was monitored with a Hewlett-Packard recording spectrophotometer for three minutes. This process was practiced for each of the thirteen samples and for a "NERL" bicarbonate standard, obtained from New England Reagent Laboratory, East

Providence, R.I., having a known bicarbonate content of 25 meq/l. The working reagent was also preincubated and its rate of decrease in absorbance was identically determined (the "blank"). The change in absorbance at three 30-second fixed-time intervals was averaged to obtain a mean change in absorbance for each of the thirteen serum samples, the standard and the blank.

The concentration of bicarbonate in each of the serial serum samples was determined algebraically as the direct ratio of the average change in absorbance at 30-second intervals for the bicarbonate standard, taking into account, conventionally, the average change in absorbance of the blank over the 30-second interval as follows:

$$HCO_3^- \text{ conc.} = \frac{(\Delta A/30 \text{ sec. sample} - \Delta A/30 \text{ sec. blank})\ (\text{standard conc.})}{\Delta A/30 \text{ sec. standard} - \Delta A/30 \text{ sec. blank}}$$

where concentration is in meq/l and "A/30 sec." indicates the average change in absorbance at fixed-time intervals of 30 seconds. The ΔA/30 sec. for the blank, the standard and the serial samples, and the determined bicarbonate recovery compared to the known bicarbonate content of the serial samples, are set forth in Table I:

TABLE I

| Sample | ΔA/30 sec. | Bicarbonate (meq/l) | |
|---|---|---|---|
| | | Determined | Expected |
| blank | 0.026 | | |
| standard (25 meq/l) | 0.079 | | |
| Serum 1 | 0.031 | 2.4 | 3.0 |
| Serum 2 | 0.040 | 6.6 | 6.2 |
| Serum 3 | 0.046 | 9.4 | 9.4 |
| Serum 4 | 0.051 | 11.8 | 12.6 |
| Serum 5 | 0.059 | 15.6 | 15.8 |
| Serum 6 | 0.068 | 19.8 | 19.0 |
| Serum 7 | 0.074 | 22.6 | 22.1 |
| Serum 8 | 0.082 | 26.4 | 25.3 |
| Serum 9 | 0.086 | 28.3 | 28.5 |
| Serum 10 | 0.094 | 32.1 | 31.7 |
| Serum 11 | 0.101 | 35.4 | 34.9 |
| Serum 12 | 0.106 | 37.7 | 38.0 |
| Serum 13 | 0.112 | 40.6 | 41.2 |

Regression analysis of the determined bicarbonate content (y-axis) against the known bicarbonate content of the serial serum samples (x-axis) for the data in Table I yielded the following linear parameters: slope, 1.004; intercept, 0.016; and a correlation coefficient of 0.999, indicating very good linearity.

Example II

The within-day precision of the kinetic method described in Example I was calculated using three human serum pools in 18 replicates in random order. For a serum pool mean bicarbonate content of 14.7 meq/l, the coefficient of variation was 4.0%; for a serum pool mean of 24.3 meq/l, the coefficient of variation was 3.8%; and for a serum pool mean of 38.7 meq/l, the coefficient of variation was 2.3%, indicating very good precision.

Example III

Correlation of the kinetic method of Example I to an end point procedure was tested. Another set of serum samples, obtained by serially diluting serum having a known sodium bicarbonate content, was tested for bicarbonate concentration both according to the kinetic method described in Example I and according to an end point method in which a reagent kit included a substrate-buffer component containing in lyophilized form

| | |
|---|---|
| PEP | 6.4 mmol/l |
| NADH | 1.2 mmol/l |
| Magnesium acetate | 3.0 mmol/l |
| Tris-base (pH 8.0) | 100 mmol/l |
| sodium oxamate | 20 mmol/l |

(the sources of these compounds were the same as the sources stated in Example I) and an enzyme suspension containing

| | |
|---|---|
| PEPC (wheat germ) | 1 U/ml |
| MDH (porcine heart) | 5 U/ml |

The PEPC and MDH were obtained from Boehringer Mannheim Corporation, Indianapolis, Indiana.

In the end point method, 0.8 milliliters of the reconstituted reagent was combined with 0.8 ml of the enzymes suspension to form 1.6 ml of a working reagent. Two such working reagents were prepared, one to serve as a "blank". Each working reagent was preincubated at 37°C until temperature equilibrium was established, and 7.5 microliters of sample was added to one of them to form the reaction mixture. The reaction mixture and the blank were maintained at 37°C for five minutes, at which time reaction equilibrium was achieved. Net absorbance change between the reaction mixture and the blank was recorded at 340 nm in a 0.5 cm cuvette. Concentration results were calculated using the NADH extinction coefficient.

The comparative results on testing the serial serum samples by the kinetic method described in Example 1 and the end-point method of this Example II are set forth in Table II.

TABLE II

| Sample # | Bicarbonate (meq/l)<br>Determined kinetically | Determined by an end-point method |
|---|---|---|
| 1 | 4.8 | 5.7 |
| 2 | 8.3 | 7.7 |
| 3 | 10.2 | 11.8 |
| 4 | 14.2 | 14.7 |
| 5 | 16.1 | 17.0 |
| 6 | 19.1 | 21.5 |
| 7 | 22.9 | 23.3 |
| 8 | 27.2 | 28.2 |
| 9 | 30.5 | 31.5 |
| 10 | 37.3 | 36.2 |
| 11 | 38.8 | 39.1 |

Regression analysis of the data of Table II, in which the y-axis was the bicarbonate concentration of the serial serum samples determined by the kinetic method described in Example I and the x-axis was the

bicarbonate concentration of the same samples determined by the end-point method described under this Example II, yielded the linear parameters, slope, 1.02; intercept, −1.0; and correlation coefficient, 0.997, indicating very good correspondence to results reached by the end-point method.

Example IV

Using the same materials and methodology of the end-point determination described in Example III, the effectiveness of oxamate as an inhibitor of reaction III was determined. First, reaction III was followed by omitting the enzymes suspension component of the working reagent and adding 2 mmol/l of pyruvate and various concentrations of oxamate to serum with normal and supranormal LDH activities up to 2000 U/l (one U/l is an enzyme activity unit which catalyzes the transformation of one micromole per liter of pyruvate per minute at 37°C). In order to magnify the spectrophotometric signal, the sample volume was increased from 7.5 microliters to 50 microliters. The oxidation of NADH was followed for 10 minutes at 340 nm and 37°C, and the concentration of NADH oxidized was calculated from the change in absorbance over the 10 minute period. The results follow:

TABLE III-A

| | NADH oxidized[a], µmol/l in the reagent | | | | |
|---|---|---|---|---|---|
| LDH, U/l | No oxamate | 2.5 mmol/l oxamate | 5 mmol/l oxamate | 10 mmol/l oxamate | 20 mmol/l oxamate |
| normal[b] | 12.1 | 1.4 | 0.5 | 0.3 | 0 |
| 282 | 10.0 | 0 | 0 | 0 | 0 |
| 334 | 18.3 | 1.3 | 0.3 | 0 | 0 |
| 340 | 18.7 | 1.3 | 0 | 0 | 0 |
| 402 | 17.1 | 0 | 0 | 0 | 0 |
| 508 | 22.2 | 3.5 | 0.3 | 0.1 | 0 |
| 600 | 23.2 | 2.6 | 0.2 | 0 | 0 |
| 1200 | 37.9 | 7.7 | 0.9 | 0.3 | 0 |
| 2000 | | | | 0 | 0 |
| 1470[c] | 13.2 | 1.0 | 0 | 0 | 0 |

[a]Each result is the average of two replicates.
[b]The results represent an average of 10 normal sera.
[c]This sample was not spiked with pyruvate.

As seen from Table III-A, without oxamate present, about 10—40 µmol/l of NADH was oxidized; with oxamate added in increasing amounts, the oxidation was correspondingly diminished. At an oxamate concentration of 10—20 mmol/l, LDH activity was completely inhibited.

Oxamate was determined to have no significant effect on either PEPC or MDH by conducting a separate enzyme assay in the presence of varying amounts of oxamate, as indicated by the following table (in which the results represent an average of three replicates):

TABLE III-B

| Oxamate mmol/l | PEPC % activity | MDH % activity |
|---|---|---|
| 0 | 100 | 100 |
| 5 | 102± 2 | 102±2 |
| 10 | 95±11 | 102±2 |
| 20 | 103± 1 | 99±1 |
| 40 | 99± 2 | 98±5 |
| 80 | 86± 4 | 92±2 |

Finally, 10 mmol/l of oxamate was incorporated in the working reagent, because a much smaller sample volume is normally used in routine bicarbonate determinations than was used to obtain the data shown in Table III-A. Serum samples with various LDH activities were assayed according to the end-point procedure described in Example III before and after adding one and two millimoles/liter of pyruvate. The results, which represent an average of two replicates, follow:

TABLE III-C

| | Bicarbonate, meq/l | |
|---|---|---|
| in serum | 1 mmol/l pyruvate | 2 mmol/l pyruvate |
| 140 | 7.16 | 6.78 |
| 600 | 6.87 | 6.97 |
| 1070 | 7.11 | 6.37 |
| 1530 | 7.21 | 7.04 |
| 2000 | 7.08 | 7.03 |

The results of Table III-C show no change in bicarbonate concentration even in the presence of elevated LDH and pyruvate. The correlation (Example III) of the rate determination performed according to my invention to the same end-point method as used in this example demonstrates the effectiveness of oxamate in improving the accuracy of rate determination for bicarbonate according to my invention.

A rate of determination of a body fluid bicarbonate according to my invention is seen, therefore, to eliminate preparation and resort to a standard curve of the rate change of NADH absorbance decrease with increasing bicarbonate concentration. With my invention the rate of change of NADH absorbance decrease with increasing bicarbonate concentration is directly proportional to the bicarbonate concentration. Thus the manufacturer of a computer-based automated analyzer need not to develop programming to relate NADH absorbance decrease readings to a standard curve, and the analyzer user as well as a manual tester user is freed from having to conduct a "run" of numerous bicarbonate standards of varied concentrations in order to generate the data for that standard curve. With my invention, only a single calibrator standard need be run. In an embodiment of my invention, falsely elevated bicarbonate rate determinations caused by body fluid endogenous lactate dehydrogenase and pyruvate are prevented by utilization of oxamate.

**Claims**

1. A reagent for the enzymatic determination of bicarbonate concentration in a body fluid by measuring the linear-rate formation of oxaloacetate from phosphoenol-pyruvate by phosphoenolpyruvate carboxylase in the presence of the bicarbonate in said body fluid which, defined with respect to the volume of a reaction mixture formed upon combining the body fluid and a reagent, comprises:

— from about 3 to about 40 mmol/l of phosphoenolpyruvate and from about 0,2 to about 0,6 mmol/l of the reduced form of nicotinamide adenine dinucleotide,
— from about 0,1 to about 0,8 U/ml of phosphoenolpyruvate carboxylase,
— from about 0,5 to about 4 U/ml of malate dehydrogenase, and
— from about 1 to about 30 mmol/l of inorganic pyrophosphate.

2. The reagent of claim 1 further comprising from about 5 to about 40 mmols of oxamate per liter of reaction mixture.

3. The reagent of claim 1 or 2 further comprising a soluble salt of magnesium.

4. The reagent of claim 3 which comprises from about 0,5 to about 5 mmol/l of a soluble magnesium salt.

5. The reagent of claims 1, 3 and 4 which further comprises a buffer having a pKa effective to provide a basic pH in the range from 7 to 9,5.

6. A method of kinetically determining the concentration of bicarbonate in a body fluid, which comprises a combining the body fluid with a reagent as claimed in one of the claims 1—5 to form a test reaction mixture

maintaining said test reaction mixture at a substantially constant temperature within the range from about 20°C to about 50°C and at a substantially constant pH within the range from about 7 to about 9.5,

measuring the decrease in absorbance of the reaction mixture at a wavelength in the range from about 320 nm to about 400 nm over a fixed-time interval while said formation of oxaloacetate is occurring at a constant rate,

forming a second reaction mixture to have the same volume as the test reaction mixture by combining said reagent with a solution having a known bicarbonate concentration,

maintaining said second reaction mixture under the same conditions of pH, temperature and time as for said test reaction mixture,

measuring the decrease in absorbance of said second reaction mixture at the same lapsed time and same fixed-time interval as for said test reaction mixture, and

applying to said measured absorbance decrease of said test reaction mixture the factor in which the numerator is said known bicarbonate concentration of said solution and in which the denominator is said measured absorbance decrease of said second reaction mixture.

7. A method as claimed in claim 6, which comprises the further steps of:

maintaining a volume of said reagent identical to said volume of said test reaction mixture at the same conditions of the temperature, pH and time as for said test reaction mixture,

measuring the decrease in absorbance of said reagent at the same wavelength and at the same fixed-time interval as for the test reaction mixture,

reducing the measured absorbance increases of each of said test reaction mixture and said second reaction mixture by the measured absorbance decrease of said reagent, and

applying to the so reduced measured absorbance decrease of said test reaction mixture the factor in which the numerator is said known bicarbonate concentration of said solution and in which the denominator is the so reduced measured absorbance decrease of said second reaction mixture.

**Patentansprüche**

1. Ein Reagenz zur enzymatischen Bestimmung der Bicarbonat-Konzentration in einer Körperflüssigkeit durch Messung der geschwindigkeitsproportionalen Bildung von Oxalacetat aus Phosphoenolpyruvat mittels Phosphoenolpyruvatcarboxylase in Gegenwart des Bicarbonats in besagter Körperflüssigkeit, dadurch gekennzeichnet, daß es im Hinblick auf das Volumen eines Reaktionsgemisches, welches durch Kombination der Körperflüssigkeit und eines Reagenzes entsteht, folgendes enthält:

— zwischen ca. 3 und ca. 40 mmol/l Phosphoenolpyruvat und zwischer ca. 0,2 und ca. 0,6 mmol/l Nicotinamidadenindinucleotids in reduzierter Form,
— zwischen ca. 0,1 und ca. 0,8 U/ml Phosphoenolpyruvatcarboxylase,
— zwischen ca. 0,5 und ca. 4 U/ml Malatdehydrogenase und
— zwischen ca. 1 und ca. 30 mmol/l anorganisches Pyrophosphat.

2. Das Reagenz gemäß Anspruch 1, dadurch gekennzeichnet, daß es außerdem zwischen ca. 5 und ca. 40 mmol Oxamat pro Liter Reaktionsgemisch enthält.

3. Das Reagenz gemäß den Ansprüchen 1 oder 2, dadurch gekennzeichnet, daß es außerdem ein lösliches Magnesiumsalz enthält.

4. Das Reagenz gemäß Anspruch 3, dadurch gekennzeichnet, daß es zwischen ca. 0,5 und ca. 5 mmol/l eines löslichen Magnesiumsalzes enthält.

5. Das Reagenz gemäß den Ansprüchen 1, 3 und 4, dadurch gekennzeichnet, daß es außerdem einen Puffer mit einem zur Erreichung eines basischen pH im Bereich zwischen 7 und 9,5 wirksamen pKa besitzt.

6. Ein Verfahren zu kinetischen Bestimmung der Bicarbonatkonzentration in einer Körperflüssigkeit, dadurch gekennzeichnet, daß die Körperflüssigkeit mit einem Reagenz gemäß den Ansprüchen 1 bis 5 zu einem zu untersuchenden Reaktionsgemisch vereint wird,

dieses zu untersuchende Reaktionsgemisch bei einer im wesentlichen konstanten Temperatur im Bereich zwischen ca. 20 und ca. 50°C und bei einem im wesentlichen konstanten pH im Bereich zwischen ca. 7 und za. 9,5 gehalten wird,

die Absorptionsabnahme der Reaktionsmichung bei einer Wellenlänge im Bereich zwischen ca. 320

und ca. 400 nm während eines festgelegten Zeitraumes, in dem besagte Bildung des Oxalacetats mit konstanter Geschwindigkeit erfolgt, gemessen wird,

eine zweite Reaktionsmischung hergestellt wird, die durch Vereinigung besagten Reagenzes mit einer Lösung mit bekannter Bicarbonatkonzentration des gleiche Volumen wie das zu untersuchende Reaktionsgemische besitzt,

für diese zweite Reaktionsmischung die gleichen Bedingungen bezüglich pH, Temperatur und Zeit eingehalten werden wie für das zu untersuchende Reaktionsgemisch,

die Absorptionsabnahme dieser zweiten Reaktionsmischung nach der gleichen Zeitspanne und im gleichen festgelegten Zeitraum wie die des zu untersuchenden Reaktionsgemisches gemessen wird und

diese gemessene Absorptionsabnahme des zu untersuchenden Reaktionsgemisches mit dem Faktor multipliziert wird, bei dem der Zähler die bekannte Bicarbonatkonzentration der Lösung und der Nenner die gemessene Absorptionsabnahme der zweiten Reaktionsmischung enthält.

7. Das Verfahren gemäß Anspruch 6, dadurch gekennzeichnet, daß es außerdem folgende Schritte enthält:

Einhaltung der gleichen Bedingungen bezüglich Temperatur, pH und Zeit für ein Volumen des Reagenzes, welches mit dem Volumen des zu untersuchenden Reaktionsgemisches übereinstimmt,

Messung der Absorptionsabnahme des Reagenzes bei der gleichen Wellenlänge und im gleichen festgelegten Zeitraum wie für das zu untersuchende Reaktionsgemisch,

Verringerung der gemessenen Absorptionszunahme sowohl des zu untersuchenden Reaktionsgemisches als auch der zweiten Reaktionsmischung um die gemessene Absorptionsabnahme des Reagenzes und

Multiplikation der so verringerten, gemessenen Absorptionsabnahme des zu untersuchenden Reaktionsgemisches mit dem Faktor, der im Zähler die bekannte Bicarbonatkonzentration besagter Lösung und im Nenner die so verringerte, gemessene Absorptionsabnahme der zweiten Reaktionsmischung enthält.

**Revendications**

1. Réactif pour la détermination par voie enzymatique de la concentration de bicarbonate dans un liquide somatique au moyen d'une mesure de la formation à vitesse linéaire d'oxalo-acétate à partir de phosphoénolpyruvate avec du phosphoénolpyruvate-carboxylase en présence du bicarbonate dans le liquide somatique en question, le réactif comprenant, relativement au volume d'un mélange réactionnel formé en réunissant le liquide somatique et un réactif:

— entre environ 3 et environ 40 mmoles/l de phosphoénolpyruvate et entre environ 0,2 et environ 0,6 mmole/l de la forme réduite de nicotinamideadénine dinucléotide,
— entre environ 0,1 et environ 0,8 U/ml de phosphoénolpyruvate-carboxylase,
— entre environ 0,5 et environ 4 U/ml de malate-déshydrogénase, et
— entre environ 1 et environ 30 mmoles/l de pyrophosphate minéral.

2. Le réactif selon la revendication 1 comprenant en outre entre environ 5 et environ 40 mmoles d'oxamate par litre de mélange réactionnel.

3. Le réactif selon la revendication 1 ou 2 comprenant en outre un sel de magnésium soluble.

4. Le réactif selon la revendication 3 comprenant entre environ 0,5 et environ 5 mmoles/l d'un sel de magnésium soluble.

5. Le réactif selon les revendications 1, 3 et 4 comprenant en outre un tampon ayant un pKa susceptible de pourvoir à un pH basique compris entre 7 et 9,5.

6. Procédé pour déterminer par voie cinétique la concentration de bicarbonate dans un liquide somatique, dans lequel on réunit le liquide somatique avec un réactif tel que défini dans l'une quelconque des revendications 1 à 5 afin de former un mélange réactionnel,

on maintient ce mélange réactionnel d'essai à une température essentiellement constante comprise entre environ 20°C et environ 50°C et à un pH pratiquement constant compris entre environ 7 et environ 9,5,

on mesure la diminution du pouvoir absorbant du mélange réactionnel à une longueur d'onde comprise entre environ 320 nm et environ 400 nm pendant un intervalle de temps fixe tandis que la formation d'oxalo-acétate ci-dessus évoquée se déroule à une vitesse constante,

on forme un second mélange réactionnel ayant le même volume que le mélange réactionnel d'essai en réunissant le présent réactif avec une solution ayant une concentration connue en bicarbonate,

on maintient le second mélange réactionnel dans les mêmes conditions de pH, de température et de durée que le mélange réactionnel d'essai,

on mesure la diminution du pouvoir absorbant du second mélange réactionnel pendant le même temps écoulé et le même intervalle de temps fixe que pour le mélange réactionnel d'essai, et

on multiple la diminution mesurée du pouvoir absorbant du mélange réactionnel d'essai par un facteur dans lequel le numérateur est la concentration de bicarbonate connue de la solution et dans lequel le dénominateur est la diminution mesurée du pouvoir absorbant du second mélange réactionnel.

7. Procédé selon la revendication 6, comprenant en outre les stades suivants:

on maintient un volume du présent réactif identique au volume du mélange réactionnel d'essai dans les mêmes conditions de température, de pH et de durée que le mélange réactionnel d'essai,

on mesure la diminution du pouvoir absorbant du réactif à la même longueur d'onde et pendant le même intervalle de temps fixe que pour le mélange réactionnel d'essai,

on réduit les augmentations mesurées du pouvoir absorbant du mélange réactionnel d'essai et du second mélange réactionnel, de la diminution du pouvoir absorbant mesurée de ce réactif et on multiplie la diminution mesurée du pouvoir absorbant ainsi abaissée du mélange réactionnel d'essai par le facteur dans lequel le numérateur est la concentration connue du bicarbonate de la solution et dans lequel le dénominateur est la diminution mesurée du pouvoir absorbant ainsi abaissée du second mélange réactionnel.